# EUROPEAN PATENT SPECIFICATION

(11) **EP 2 389 166 B1**
(45) Date of publication and mention of the grant of the patent: **21.11.2012**
(21) Application number: 10701340.1
(22) Date of filing: 13.01.2010
(51) Int. Cl.: A61K 9/20, A61K 31/422

(54) **PHARMACEUTICAL COMPOSITION COMPRISING ALEGLITAZAR**
PHARMAZEUTISCHE ZUSAMMENSETZUNG MIT ALEGLITAZAR
COMPOSITION PHARMACEUTIQUE COMPRENANT DE L'ALEGLITAZAR

(30) Priority: 23.01.2009 EP 09151254
(43) Date of publication of application: 30.11.2011
(73) Proprietor: F. Hoffmann-La Roche AG, 4070 Basel (CH)
(72) Inventor: GLOMME, Alexander, CH-4127 Birsfelden (CH); WOJTERA, Paul, CH-4123 Allschwil (CH)
(74) Representative: Pomeranc, Didier
(86) International application number: PCT/EP2010/050343
(87) International publication number: WO 2010/084066

(56) References cited:
- WO-A1-02/092084
- WO-A1-2007/068577
- WO-A1-2008/119070
- WO-A2-2004/073627
- US-A1- 2006 270 722

## Description

The invention relates to a pharmaceutical composition and in particular to a pharmaceutical composition comprising aleglitazar or a salt thereof. More particularly, the invention relates to a pharmaceutical composition comprising 0.01 mg to 0.9 mg of aleglitazar or a salt thereof obtainable by
(a) spraying a solution comprising aleglitazar or a salt thereof on a diluent and a disintegrant;
(b) mixing the composition obtained in step (a) with a lubricant; and
(c) optionally compressing the composition obtained in step (b).

The invention also relates to a process for the manufacture of a pharmaceutical composition comprising 0.01 mg to 0.9 mg of aleglitazar or a salt thereof comprising:
(a) spraying a solution comprising aleglitazar or a salt thereof on a diluent and a disintegrant;
(b) mixing the composition obtained in step (a) with a lubricant; and
(c) optionally compressing the composition obtained in step (b).

Aleglitazar is (S)-2-methoxy-3-{4-[2-(5-methyl-2-phenyl-oxazol-4-yl)-ethoxy]-benzo[b]thiophen-7-yl}-propionic acid. It belongs to the class of Peroxisome Proliferator Activated Receptors (PPAR) agonists. Aleglitazar is described in WO 02/092084. The sodium salt of aleglitazar is sodium (S)-2-methoxy-3-{4-[2-(5-methyl-2-phenyl-oxazol-4-yl)-ethoxy]-benzo[b]thiophen-7-yl}-propanoate.

Peroxisome Proliferator Activated Receptors are members of the nuclear hormone receptor super family, which are ligand-activated transcription factors regulating gene expression. Various subtypes thereof have been identified and cloned. These include PPARα, PPARβ (also known as PPARδ), and PPARγ. There exist at least two major isoforms of PPARγ. While PPARγ1 is ubiquitously expressed in most tissues, the longer isoform PPARγ2 is almost exclusively found in adipocytes. In contrast, PPARα is predominantly expressed in the liver, kidney and heart. PPAR's modulate a variety of body responses including glucose- and lipid- homeostasis, cell differentiation, inflammatory responses and cardiovascular events.

Studies provide evidence that a coagonism on PPARα and PPARγ results in compounds with enhanced therapeutic potential, i. e. with an improved lipid profile effect on top of the normalization of glucose- and insulin-levels (Keller and Wahli: Trends Endocrin. Metab. 1993; 4:291-296, Macdonald and Lane: Current Biology Vol.5 pp.618-621 (1995)).

Aleglitazar is capable of binding and activating both PPARα and PPARγ simultaneously and very efficiently. Therefore, aleglitazar combines the anti-glycemic effect of PPARγ activation with the anti-dyslipidemic effect of PPARα activation. Consequently, plasma glucose and insulin are reduced (=insulin sensitization), triglycerides lowered and HDL cholesterol increased (=improved lipid profile). In addition, it lowers LDL cholesterol, decrease blood pressure and counteract inflammatory atherosclerosis. Since multiple facets of the type II diabetes disease syndrome are addressed by PPARα and γ coagonists, Aleglitazar has an enhanced therapeutic effect compared to the compounds already known in the art.

It was surprisingly found that a pharmaceutical composition comprising aleglitazar in a dose range of 0.01 to 0.9 mg is particularly efficient in treating or preventing diabetes type II. Due to a particularly low solubility and a high activity, aleglitazar had never been successfully included in a homogenous low dose solid formulation suitable for commercial and industrial processes.

In the present description the term "diluent" refers to an excipient which fills out the size of a tablet or capsule, making it practical to produce and convenient for the consumer to use. Suitable diluents include e.g. pharmaceutically acceptable inert fillers, such as microcrystalline cellulose, lactose, dibasic calcium phosphate sugar, sugar alcohols, corn starch, sucrose, silicic anhydride, polysaccharides, N-methyl pyrrolidone (Pharmasolve (ISP)) and mixtures thereof. The term sugar and sugar alcohols comprises mannitol, lactose, fructose, sorbitol, xylitol, maltodextrin, dextrates, dextrins, lactitol and mixtures thereof.

Binders are added to tablet formulations to add cohesiveness to powders thereby providing the necessary bonding to form granules which under compaction form a compact mass as tablet. Methyl cellulose, carboxymethylcellulose, hydroxypropylcellulose, hydroxymethyl propylcellulose, polyvinylpyrrolidone, polyvinyl alcohol, starch and starch pregelatinized are suitable examples of binders. Mixtures of two, three or more binders can be used in the formulation.

The solution comprising aleglitazar can be an aqueous or an organic solution. The preferred organic solvent is ethanol.

Buffer solutions are used as a mean for keeping pH at a nearly constant value. A buffer solution is usually an aqueous solution consisting of a mixture of a weak acid and its conjugate base or a weak base and its conjugate acid. It has the property that the pH of the solution changes very little when a small amount of acid or base is added to it. Examples of buffers are acetic acid/sodium acetate buffer, potassium or sodium dihydrogenphosphate/dipotassium or disodium phosphate buffer, boric acid/sodium hydroxide buffer, tris (tris-(hydroxymethyl)-aminomethane)-buffer, carbonate/bicarbonate and EDTA-buffer.

The solution comprising aleglitazar is preferably a buffered aqueous solution, preferably buffered with a phosphate buffer. The phosphate buffer is preferably a buffer comprising disodium phosphate and sodium dihydrogen phosphate.

The term "surfactant" refers to an excipient that lowers the surface tension of a liquid. Examples of surfactant include tween 80, polyoxyethylene-polyoxypropylene copolymer and sodium lauryl sulfate.

The term "disintegrant" refers to an excipient which expands and dissolve when wet causing the tablet to break apart in the digestive tract, releasing the active ingredients for absorption. Suitable disintegrants include e.g. lightly crosslinked polyvinyl pyrrolidone, corn starch, potato starch, maize starch, and modified starches, croscarmellose sodium, carboxymethylcellulose calcium, carboxymethylcellulose sodium, crossprovidone, sodium starch glycolate and mixtures thereof.

Suitable lubricants, including agents that act on the flowability of the powder by reducing interparticle friction and cohesion to be compressed, are colloidal silicon dioxide, such as aerosil, talc, stearic acid, magnesium stearate, calcium stearate, glyceryl behenate, sodium stearyl fumarate and silica gel.

The term "coating" refers to an excipient which is applied on the surface of a tablet and which protects tablet ingredients from deterioration by moisture in the air and make large or unpleasant-tasting tablets easier to swallow. Examples of coating agent include PVA (polyvinyl alcohol), HPMC (hydroxypropylmethyl cellulose) and PEG (polyethylene glycol).

The term "aleglitazar or a salt thereof' refers to aleglitazar or to the reaction product of aleglitazar with a base wherein the carboxylic acid hydrogen atom of aleglitazar has been replaced by a metal atom. A preferred salt of aleglitazar is the sodium salt of aleglitazar.

Unless otherwise stated all percentages are given in weight percent of the total weight of the composition.

The weight range of aleglitazar or the salt thereof refers to the weight of aleglitazar in the form of the free acid or, in case of the salt, to the weight of the same mole quantity of aleglitazar in the form of the free acid.

The amount of aleglitazar or the salt thereof in the pharmaceutical composition or in the process is between 0.01 and 0.9 mg, preferably between 0.01 and 0.6 mg, more preferably between 0.15 and 0.6 mg. Further preferred amounts of aleglitazar are 0.15 or 0.3 mg.

Preferred is a pharmaceutical composition or a process, wherein the solution comprising aleglitazar or a salt thereof in step (a) is an aqueous solution.

Further preferred is a pharmaceutical composition or a process, wherein the solution comprising aleglitazar or a salt thereof in step (a) is an organic solution, preferably an ethanolic solution.

Preferred is a pharmaceutical composition or a process according to the invention, wherein the solution of step (a) is an aqueous solution, preferably buffered with a phosphate buffer. The buffer is preferably selected from the buffers mentioned above and is more preferably disodium phosphate and sodium dihydrogen phosphate.

When the buffer is Na₂HPO₄ and NaH₂PO₄ the molarity of the buffer system is preferably ranging from 0.05 to 0.4 mM, preferably from 0.1 to 0.2 mM. The pH of the buffered solution is preferably ranging between 6.0 and 12.

Further preferred is a pharmaceutical composition or a process according to the invention, wherein the diluent is selected from sugar, microcrystalline cellulose, lactose, starch and mixtures thereof and is preferably a mixture of lactose and microcrystalline cellulose.

Also preferred is a pharmaceutical composition or a process according to the invention, wherein the aqueous solution of step (a) comprises a binder selected from povidone, HPMC and starch pregelatinized.

The amount of binder is preferably between 1 and 15 %, more preferably between 2 and 10 %, more preferably between 4 and 6 % of the total weight of the composition.

The binder is preferably povidone. Starch pregelatinized is further preferred.

Preferred is a pharmaceutical composition or a process according to the invention, wherein the solution of step (a) comprises a surfactant selected from tween 80, polyoxyethylene-polyoxypropylene copolymer and sodium lauryl sulfate.

The amount of surfactant is preferably between 0.1 and 2 %, more preferably between 0.25 and 1 %, more preferably between 0.4 and 0.6 % of the total weight of the composition.

The surfactant is preferably sodium lauryl sulfate.

The disintegrant is preferably selected from crosslinked polyvinyl pyrrolidone, starch, croscarmellose sodium, carboxymethylcellulose sodium and sodium starch glycolate.

The amount of disintegrant is preferably between 0.5 and 15 %, more preferably between 1 and 10 %, more preferably between 2 and 3 % of the total weight of the composition.

The disintegrant is preferably croscarmellose sodium.

Also preferred is a pharmaceutical composition or a process according to the invention, wherein the lubricant is selected from stearic acid, magnesium stearate, glyceryl behenate and sodium stearyl fumarate.

The amount of lubricant is preferably between 0.2 and 3 %, more preferably between 0.5 and 1.0 % of the total weight of the composition.

The lubricant is preferably magnesium stearate.

The composition preferably comprises a sodium salt of aleglitazar.

The invention also preferably relates to a pharmaceutical composition comprising:

| | |
|---|---|
| Aleglitazar or sodium salt thereof | 0.01 - 2% |
| Disodium phosphate | 0.5 - 1.5 % |
| Sodium dihydrogen phosphate | 0.005 - 0.25 % |
| Sodium lauryl sulfate | 0.1 - 2% |
| Povidone or HPMC | 1 - 15 % |
| Lactose | 15 - 85% |
| Microcrystalline cellulose | 3 - 85% |
| Croscarmellose sodium | 0.5 - 15% |
| Magnesium stearate | 0.2 - 3%. |

The invention also preferably relates to a pharmaceutical composition comprising:

| | |
|---|---|
| Aleglitazar or sodium salt thereof | 0.01 - 0.9 mg |
| Disodium phosphate | 1 - 1.5 mg |
| Sodium dihydrogen phosphate | 0.01 - 0.3 mg |
| Sodium lauryl sulfate | 0.1 - 2 mg |
| Povidone or HPMC | 0.1 - 20 mg |
| Lactose | 20 - 110mg |
| Microcrystalline cellulose | 5 - 100 mg |
| Croscarmellose sodium | 0.5 - 20 mg |
| Magnesium stearate | 0.5 - 3 mg. |

If desired, the pharmaceutical compositions of the present invention may also contain one or more antioxidants in order to prevent e.g. any oxidation of the drug compound. Suitable antioxidants for use herein include butylated hydroxyanisole, sodium ascorbate, butylated hydroxytoluene, sodium metabisulfate, malic acid, citric acid, ascorbic acid and mixtures thereof. A preferred antioxidant is butylated hydroxyanisole and butylated hydroxytoluene in a mixture of 1:1.

Furthermore, the invention also relates to a pharmaceutical composition comprising:

| | |
|---|---|
| Aleglitazar | 0.01 - 2% |
| Butylated Hydroxyanisole | 0.01 - 0.5% |
| Butylated Hydroxytoluene | 0.01 - 0.5% |
| Sodium lauryl sulfate | 0.1 - 2% |
| Povidone | 1 - 15 % |
| Lactose | 15 - 85% |
| Microcrystalline cellulose | 3-85% |
| Croscarmellose sodium | 0.5-15% |
| Magnesium stearate | 0.2-3%. |

The invention also relates to a pharmaceutical composition comprising:

| | |
|---|---|
| Aleglitazar | 0.01 - 0.9 mg |
| Butylated Hydroxyanisole | 0.01 - 0.5 mg |
| Butylated Hydroxytoluene | 0.01 - 0.5 mg |
| Sodium lauryl sulfate | 0.1 - 2 mg |
| Povidone | 0.1 - 20 mg |
| Lactose | 20 - 110 mg |
| Microcrystalline cellulose | 5 - 100 mg |
| Croscarmellose sodium | 0.5 - 20 mg |
| Magnesium stearate | 0.5 - 3 mg. |

The above composition, comprising an anti-oxidant, is preferred when an ethanolic solution is used in step (a).

The pharmaceutical composition of the invention can be in the form of a tablet or a capsule and is preferably in the form of a tablet.

The preferred weight of the tablet is between 50 and 250 mg, preferably between 60 and 200mg, more preferably between 70 and 150 mg. A particularly preferred weight is 130 mg.

The pharmaceutical composition of the invention can be coated with any suitable coating known in the art, preferably for example with Opadry II white.

The invention also relates to a pharmaceutical composition as described above for use as medicament, and preferably for use as medicament for the treatment or prophylaxis of diabetes type II or cardiovascular diseases.

Aleglitazar is particularly indicated for the reduction of cardiovascular mortality, non-fatal myocardial infarction or stroke, in particular in patients having stable coronary heart syndrome and diabetes type II.

For the preparation of the composition the following procedure can be followed.

A buffered solution is prepared, comprising of water, aleglitazar, buffer salts, binder and surfactant. Aleglitazar is preferably in the amount of 0.2 to 0.8 % (m/v). The active is preferably completely dissolved in the granulation liquid. The diluents and the disintegrant are added to a fluid bed granulator. The solution is uniformly sprayed on to the excipients to archieve granules with excellent content uniformity for these low dosage strengths and an appropiate particle size distribution (d63.2 is preferably in the range from 0.15 to 0.8 mm (mesh size through which pass 63.2% of the granules)) to avoid high potent dust exposure and obtain good flowability properties. The granulate is preferably dried afterwards in the fluid bed dryer. This is preferably followed by sieving of the resultant granules through a mesh screen to obtain the fluid bed granules (FBG). The dried granules are preferably blended with the lubricant and then pressed into tablets using conventional tablet pressing equipment. The tablets of the invention may take any appropriate shape, such as discoid, round, oval, oblong, cylindrical, triangular, hexagonal, and the like. The drug load in the kernels is ranging from 0.01 to 0.3 %, preferably from 0.05 to 0.15 %. Tablet hardness is preferably above 60 N to assure good coating and packaging properties. The tablets have preferably an oval shape to allow good swallow and handling properties. All of the tablets showed rapid disintegration (less than 300 seconds, aqueous medium, standard basket method and equipment). The pressed tablets can be film coated with a coating inducing a 4% weight increase.

The invention is illustrated hereinafter by Examples.

### Examples

### Example 1: tablet

| | |
|---|---|
| Aleglitazar or sodium salt thereof | 0.3 mg |
| Disodium phosphate | 1.106 mg |
| Sodium dihydrogen phosphate | 0.037 mg |
| Sodium lauryl sulfate | 0.625 mg |
| HPMC | 6.25 mg |
| Lactose | 100.43 mg |
| Microcrystalline cellulose | 12.5 mg |
| Croscarmellose sodium | 2.5 mg |
| Magnesium stearate | 1.25 mg |
| Opadry II White | 5.00 mg. |

A buffered aqueous solution comprising alglitazar (0.3 % (w/v)), disodium phosphate, sodium dihydrogen phosphate, HPMC and sodium lauryl sulfate was prepared. The active was completely dissolved in the granulation liquid. Agitation was required in order to achieve complete disolution. Lactose, microcrystalline cellulose and croscarmellose sodium were added to a fluid bed granulator. The solution was uniformly sprayed (spraying rate between 180 - 220 g/min; inlet air temperature 60 to 80 °C, air pressure 2 to 4 bar, product temperature 22°C to 35°C) on to the solid excipients to archieve granules. The obtained granules hd excellent content uniformity for this low dosage strength and an appropiate particle size distribution (d63.2 is 250 µm) hereby avoiding high potent dust exposure. Moreover, the granules had good flowability properties. The granulate was dried afterwards in the fluid bed dryer. This was followed by sieving of the resultant granules through a mesh screen (2 mm sieve size, 1000 rpm rotation speed) to obtain the fluid bed granules (FBG). The dried granules were blended with the lubricant and then pressed into tablets using conventional tablet pressing equipment.

### Example 2: tablet

| | |
|---|---|
| Aleglitazar or sodium salt thereof | 0.3 mg |
| Disodium phosphate | 1.106 mg |
| Sodium dihydrogen phosphate | 0.037 mg |
| Sodium lauryl sulfate | 0.625 mg |
| Povidone | 6.25 mg |
| Lactose | 100.43 mg |
| Microcrystalline cellulose | 12.5 mg |
| Croscarmellose sodium | 2.5 mg |
| Magnesium stearate | 1.25 mg |
| Opadry II White | 5.00 mg. |

The procedure described in Example 1 was repeated here with povidone instead of HPMC. The granules had content uniformity, size distribution and flowability properties similar to those of the granules of Example 1. The dried granules were blended with the lubricant and then pressed into tablets using conventional tablet pressing equipment.

### Example 3: tablet

| | |
|---|---|
| Aleglitazar | 0.15 mg |
| Butylated Hydroxyanisole | 0.0125 mg |
| Butylated Hydroxytoluene | 0.0125 mg |
| Sodium lauryl sulfate | 0.625 mg |
| Povidone | 6.25 mg |
| Lactose | 101.7 mg |
| Microcrystalline cellulose | 12.5 mg |
| Croscarmellose sodium | 2.5 mg |
| Magnesium stearate | 1.25 mg |
| Opadry II White | 5.00 mg. |

An ethanolic solution comprising aleglitazar, butylated hydroxyanisole, butylated hydroxytoluene, povidone and sodium lauryl sulfate was prepared. The active was completely dissolved in the granulation liquid. Lactose, microcrystalline cellulose and croscarmellose sodium were added to a fluid bed granulator. The solution was uniformly sprayed (200 to 400 g/min) on to the solid excipients to archieve granules. The granules had content uniformity, size distribution and flowability properties similar to those of the granules of Examples 1 and 2. The dried granules were blended with the lubricant and then pressed into tablets using conventional tablet pressing equipment.

## Claims

1. Pharmaceutical composition comprising 0.01 mg to 0.9 mg of aleglitazar or a salt thereof obtainable by
(a) spraying a solution comprising aleglitazar or a salt thereof on a diluent and a disintegrant;
(b) mixing the composition obtained in step (a) with a lubricant; and
(c) optionally compressing the composition obtained in step (b).

2. Process for the manufacture of a pharmaceutical composition comprising 0.01 mg to 0.9 mg of aleglitazar or a salt thereof comprising:
(a) spraying a solution comprising aleglitazar or a salt thereof on a diluent and a disintegrant;
(b) mixing the composition obtained in step (a) with a lubricant; and
(c) optionally compressing the composition obtained in step (b).

3. Pharmaceutical composition or process according to claim 1 or 2, wherein the solution of step (a) is an aqueous solution.

4. Pharmaceutical composition or process according to any one of claim 1 to 3, wherein the solution of step (a) is buffered with a phosphate buffer.

5. Pharmaceutical composition or process according to any one of claim 1 to 4, wherein the diluent is selected from sugar, microcrystalline cellulose, lactose, starch and mixtures thereof.

6. Pharmaceutical composition or process according to any one of claims 1 to 5, wherein the solution of step (a) comprises a binder selected from povidone, HPMC and starch pregelatinized.

7. Pharmaceutical composition or process according to claim 6, wherein the binder is povidone.

8. Pharmaceutical composition or process according to any one of claims 1 to 7, wherein the solution of step (a) comprises a surfactant selected from tween 80, polyoxyethylene-polyoxypropylene copolymer and sodium lauryl sulfate.

9. Pharmaceutical composition or process according to claim 8, wherein the surfactant is sodium lauryl sulfate.

10. Pharmaceutical composition or process according to any one of claims 1 to 9, wherein the disintegrant is selected from crosslinked polyvinyl pyrrolidone, starch, croscarmellose sodium, carboxymethylcellulose sodium and sodium starch glycolate.

11. Pharmaceutical composition or process according to claim 10, wherein the disintegrant is croscarmellose sodium.

12. Pharmaceutical composition or process according to any one of claims 1 to 11, wherein the lubricant is selected from stearic acid, magnesium stearate, glyceryl behenate and sodium stearyl fumarate.

13. Pharmaceutical composition or process according to claim 12, wherein the lubricant is magnesium stearate.

14. Pharmaceutical composition or process according to any one of claims 1 to 13, comprising a sodium salt of aleglitazar.

15. Pharmaceutical composition comprising:
| | |
|---|---|
| Aleglitazar or sodium salt thereof | 0.01 - 0.9 mg |
| Disodium phosphate | 1 - 1.5 mg |
| Sodium dihydrogen phosphate | 0.01 - 0.3 mg |
| Sodium lauryl sulfate | 0.1 - 2 mg |
| Povidone | 0.1 - 20 mg |
| Lactose | 20 - 110 mg |
| Microcrystalline cellulose | 5 - 100 mg |
| Croscarmellose sodium | 0.5 - 20 mg |
| Magnesium stearate | 0.5 - 3 mg. |

16. Pharmaceutical composition according to any one of claims 1 or 3 to 15 in the form of a tablet.

17. Pharmaceutical composition according to any one of claims 1 or 3 to 16 for use as medicament for the treatment or prophylaxis of diabetes type II or cardiovascular diseases.

## Patentansprüche

1. Pharmazeutische Zusammensetzung umfassend 0,01 mg bis 0,9 mg Aleglitazar oder ein Salz davon, welche erhältlich ist durch
(a) Sprühern einer Lösung, umfassend Aleglitazar oder ein Salz davon, auf ein Streckmittel und ein Sprengmittel;
(b) Mischen der in Schritt (a) erhaltenen Zusammensetzung mit einem Gleitmittel; und
(c) gegebenenfalls Verdichten der in Schritt (b) erhaltenen Zusammensetzung.

2. Verfahren zur Herstellung einer pharmazeutischen Zusammensetzung, umfassend 0,01 mg bis 0,9 mg Aleglitazar oder ein Salz davon, welches umfasst:
(a) Sprühen einer Lösung, umfassend Aleglitazar oder ein Salz davon, auf ein Streckmittel und ein Sprengmittel;
(b) Mischen der in Schritt (a) erhaltenen Zusammensetzung mit einem Gleitmittel; und
(c) gegebenenfalls Verdichten der in Schritt (b) erhaltenen Zusammensetzung.

3. Pharmazeutische Zusammensetzung oder Verfahren gemäß Anspruch 1 oder 2, wobei die Lösung des Schritts (a) eine wässrige Lösung ist.

4. Pharmazeutische Zusammensetzung oder Verfahren gemäß einem der Ansprüche 1 bis 3, wobei die Lösung des Schritts (a) mit einem Phosphatpuffer gepuffert ist.

5. Pharmazeutische Zusammensetzung oder Verfahren gemäß einem der Ansprüche 1 bis 4, wobei das Streckmittel ausgewählt ist aus Zucker, mikrokristalliner Cellulose, Laktose, Stärke und Gemische davon.

6. Pharmazeutische Zusammensetzung oder Verfahren gemäß einem der Ansprüche 1 bis 5, wobei die Lösung des Schritts (a) ein Bindemittel, ausgewählt aus Povidon, HPMC und vorverkleisterte Stärke umfasst.

7. Pharmazeutische Zusammensetzung oder Verfahren gemäß Anspruch 6, wobei das Bindemittel Povidon ist.

8. Pharmazeutische Zusammensetzung oder Verfahren gemäß einem der Ansprüche 1 bis 7, wobei die Lösung des Schritts (a) ein oberflächenaktives Mittel, ausgewählt aus Tween 80, einem Polyoxyethylen-Polyoxypropylen Copolymer und Natriumlaurylsulfat umfasst.

9. Pharmazeutische Zusammensetzung oder Verfahren gemäß Anspruch 8, wobei das oberflächenaktive Mittel Natriumlaurylsulfat ist.

10. Pharmazeutische Zusammensetzung oder Verfahren gemäß einem der Ansprüche 1 bis 9, wobei das Sprengmittel ausgewählt ist aus quervernetztem Polyvinylpyrrolidon, Stärke, Croscarmellose-Natrium, Natriumcarboxymethylcellulose und Natriumstärkeglycolat.

11. Pharmazeutische Zusammensetzung oder Verfahren gemäß Anspruch 10, wobei das Sprengmittel Croscarmellose-Natrium ist.

12. Pharmazeutische Zusammensetzung oder Verfahren gemäß einem der Ansprüche 1 bis 11, wobei das Gleitmittel ausgewählt ist aus Stearinsäure, Magnesiumstearat, Glycerolbehenat und Natriumstearylfumarat.

13. Pharmazeutische Zusammensetzung oder Verfahren gemäß Anspruch 12, wobei das Gleitmittel Magnesiumstearat ist.

14. Pharmazeutische Zusammensetzung oder Verfahren gemäß einem der Ansprüche 1 bis 13, umfassend ein Natriumsalz von Aleglitazar,

15. Pharmazeutische Zusammensetzung, umfassend:
| | |
|---|---|
| Aleglitazar oder ein Natriumsalz davon | 0,01 - 0,9 mg |
| Dinatriumphosphat | 1 - 1,5 mg |
| Natriumdihydrogenphosphat | 0,01 - 0,3 mg |
| Natriumlaurylsulfat | 0,1 - 2 mg |
| Povidon | 0,1 - 20 mg |
| Laktose | 20 - 110 mg |
| Mikrokristalline Cellulose | 5 - 100 mg |
| Croscarmellose-Natrium | 0,5 - 20 mg |
| Magnesiumstearat | 0,5 - 3 mg |

16. Pharmazeutische Zusammensetzung gemäß einem der Ansprüche 1 oder 3 bis 15 in der Form einer Tablette.

17. Pharmazeutische Zusammensetzung gemäß einem der Ansprüche 1 oder 3 bis 16 zur Verwendung als Medikament zur Behandlung oder Vorbeugung von Typ-2-Diabetes oder cardiovaskulären Erkrankungen.

## Revendications

1. Composition pharmaceutique comprenant 0,01 mg à 0,9 mg d'aléglitazar ou d'un de ses sels, pouvant être obtenue :
(a) en atomisant une solution comprenant de l'aléglitazar ou un de ses sels sur un diluant et un agent de délitement ;
(b) en mélangeant la composition obtenue dans l'étape (a) à un lubrifiant ; et
(c) facultativement, en comprimant la composition obtenue dans l'étape (b).

2. Procédé pour la production d'une composition pharmaceutique comprenant 0,01 mg à 0,9 mg d'aléglitazar ou d'un de ses sels, comprenant :
(a) l'atomisation d'une solution comprenant de l'aléglitazar ou un de ses sels sur un diluant et un agent de délitement ;
(b) le mélange de la composition obtenue dans l'étape (a) à un lubrifiant ; et
(c) facultativement, la compression de la composition obtenue dans l'étape (b).

3. Composition pharmaceutique ou procédé suivant la revendication 1 ou 2, dans lequel la solution de l'étape (a) est une solution aqueuse.

4. Composition pharmaceutique ou procédé suivant l'une quelconque des revendications 1 à 3, dans lequel la solution de l'étape (a) est tamponnée avec un tampon au phosphate.

5. Composition pharmaceutique ou procédé suivant l'une quelconque des revendications 1 à 4, dans lequel le diluant est choisi entre le sucre, la cellulose microcristalline, le lactose, l'amidon et leurs mélanges.

6. Composition pharmaceutique ou procédé suivant l'une quelconque des revendications 1 à 5, dans lequel la solution de l'étape (a) comprend un liant choisi entre la povidone, la HPMC et l'amidon prégélatinisé.

7. Composition pharmaceutique ou procédé suivant la revendication 6, dans lequel le liant est la povidone.

8. Composition pharmaceutique ou procédé suivant l'une quelconque des revendications 1 à 7, dans lequel la solution de l'étape (a) comprend un agent tensioactif choisi entre le Tween 80, un copolymère polyoxyéthylène-polyoxypropylène et le laurylsulfate de sodium.

9. Composition pharmaceutique ou procédé suivant la revendication 8, dans lequel l'agent tensioactif est le laurylsulfate de sodium.

10. Composition pharmaceutique ou procédé suivant l'une quelconque des revendications 1 à 9, dans lequel l'agent de délitement est choisi entre la polyvinylpyrrolidone réticulée, l'amidon, le croscarmellose sodique, la carboxyméthylcellulose sodique et le glycolate d'amidon sodique.

11. Composition pharmaceutique ou procédé suivant la revendication 10, dans lequel l'agent de délitement est le croscarmellose sodique.

12. Composition pharmaceutique ou procédé suivant l'une quelconque des revendications 1 à 11, dans lequel le lubrifiant est choisi entre l'acide stéarique, le stéarate de magnésium, le béhénate de glycéryle et le stéaryl-fumarate de sodium.

13. Composition pharmaceutique ou procédé suivant la revendication 12, dans lequel le lubrifiant est le stéarate de magnésium.

14. Composition pharmaceutique ou procédé suivant l'une quelconque des revendications 1 à 13, comprenant un sel de sodium d'aléglitazar.

15. Composition pharmaceutique comprenant :
| | |
|---|---|
| de l'aléglitazar ou son sel de sodium | 0,01 - 0,9 mg |
| du phosphate disodique | 1 - 1,5 mg |
| du dihydrogénophosphate de sodium | 0,01 - 0,3 mg |
| du laurylsulfate de sodium | 0,1 - 2 mg |
| de la povidone | 0,1 - 20 mg |
| du lactose | 20 - 110 mg |
| de la cellulose microcristalline | 5 - 100 mg |
| du croscarmellose sodique | 0,5 - 20 mg |
| du stéarate de magnésium | 0,5 - 3 mg |

16. Composition pharmaceutique suivant l'une quelconque des revendications 1 ou 3 à 15, sous forme d'un comprimé.

17. Composition pharmaceutique suivant l'une quelconque des revendications 1 ou 3 à 16, pour une utilisation comme médicament pour le traitement ou la prophylaxie du diabète du type II ou de maladies cardiovasculaires.
